# EUROPÄISCHE PATENTSCHRIFT

(11) **EP 2 776 818 B1**
(45) Veröffentlichungstag und Bekanntmachung des Hinweises auf die Patenterteilung: **27.04.2016**
(21) Anmeldenummer: 12798147.0
(22) Anmeldetag: 08.11.2012
(51) Int. Cl.: G01N 23/00

(54) **PRÜFKÖRPER UND VERFAHREN ZUR ÜBERPRÜFUNG DER ÜBERTRAGUNGSEIGENSCHAFTEN VON VOLUMENTOMOGRAPHEN**
TEST BODY AND METHOD FOR CHECKING THE TRANSMISSION PROPERTIES OF VOLUME TOMOGRAPHS
CORPS D'ESSAI ET PROCÉDÉ DE VÉRIFICATION DES PROPRIÉTÉS DE TRANSMISSION DE TOMOGRAPHES VOLUMIQUES

(30) Priorität: 08.11.2011 DE 102011117859
(43) Veröffentlichungstag der Anmeldung: 17.09.2014
(73) Patentinhaber: Technische Hochschule Köln, 50968 Köln (DE)
(72) Erfinder: BLENDL, Christian, 50129 Bergheim (DE)
(74) Vertreter: Cohausz Hannig Borkowski Wißgott
(86) Internationale Anmeldenummer: PCT/EP2012/004647
(87) Internationale Veröffentlichungsnummer: WO 2013/068118

(56) Entgegenhaltungen:
- US-A- 6 076 966
- US-B1- 6 364 529
- FRANCO L ET AL: "Blurring and MTF determination of a radiotherapy EPID using the edge spread funtion", 2004 IEEE NUCLEAR SCIENCE SYMPOSIUM CONFERENCE RECORD 16-22 OCT. 2004 ROME, ITALY, IEEE, PISCATAWAY, NJ, USA, Bd. 4, 16. Oktober 2004 (2004-10-16), Seiten 2639-2641, XP010819225, DOI: 10.1109/NSSMIC.2004.1462793 ISBN: 978-0-7803-8700-3
- BARTSCHER M ET AL: "Enhancement and Proof of Accuracy of Industrial Computed Tomography (CT) Measurements", CIRP ANNALS, ELSEVIER BV, NL, CH, FR, Bd. 56, Nr. 1, 1. Januar 2007 (2007-01-01) , Seiten 495-498, XP027151109, ISSN: 0007-8506 [gefunden am 2007-01-01]
- VOIGT J.M. ET AL: "A new phantom for image quality, geometric destortion, and HU calibration in MSCT and CBCT", SPIE PROCEEDINGS, vol. 8313, 23 February 2012 (2012-02-23), DOI: 10.1117/12.911611

## Beschreibung

Die Erfindung betrifft einen Prüfkörper, insbesondere einen Prüfkörper zur Überprüfung der Übertragungseigenschaften von Volumentomographen, insbesondere von radiologischen Volumentomographen, welche üblicherweise, jedoch nicht ausschließlich Anwendung finden in der medizinischen Diagnostik und Therapie, z.B. Radiotherapie, in der Mund-,Kiefer- und Gesichtschirurgie, in der dentalen Implantologie usw.

Die Erfindung betrifft weiterhin ein Verfahren zur Überprüfung der Übertragungseigenschaften eines Volumentomographen mit einem Prüfkörper, bei dem mit dem Volumentomographen eine Serie mehrerer Messewert-Aufnahmen von dem Prüfkörper erstellt wird und aus der Serie mittels eines Tomographie-Algorithmus, bzw. Rückprojektionsalgorithmus im Volumentomographen selbst oder in einer Auswerteeinheit ein Stapel mehrerer Schichtbilder des Prüfkörpers berechnet wird.

Unter Tomographen, insbesondere Volumentomographen im Sinne der hier beschriebenen Erfindung werden sämtliche Vorrichtungen verstanden, mit denen die Möglichkeit besteht, aus einer Serie von aufgenommenen Messwerten, insbesondere aus einer Serie von aufgenommenen Bildern, einen Stapel mehrerer Schichtbilder eines untersuchten Körpers zu berechnen unter Zuhilfenahme des genannten Tomographie-Algorithmus bzw. Rückprojektions-Algorithmus.

Dabei spielt es für den hier verwendeten Begriff des Volumentomographen mit Bezug auf die Erfindung keine Rolle, welches Messprinzip bei der Erfassung der einzelnen Messwerte oder im speziellen Fall bei der Erfassung der Bilder (z.B. bei einem Röntgen-Computer-Tomographen oder einem Magnetresonanz-Tomographen) zu Grunde gelegt wird.

Beispielsweise können Volumentomographen Röntgenstrahlen einsetzen, um aus einer Serie einzelner Röntgenbilder, beispielsweise solcher Bilder die jeweils eine zweidimensionale Projektion der Röntgenstrahlabsorption darstellen, nach der Erfassung der Bilder einen Stapel von Schichtbildern zu berechnen, wobei ein solcher Stapel eine dreidimensionale Abbildung des untersuchten Körpers darstellt. Bei einem solchen Volumentomographen werden demnach 2D-Bildsensoren eingesetzt, deren Sensorpixel die Messwerte erfassen.

Ebenso sind Magnetresonanz-Tomographen bzw. Magnetresonanz-Volumentomographen bekannt, mit denen durch Einwirkung äußerer Magnetfelder in einem zu untersuchenden Gewebe so genannte Spin-Flips erzeugt werden können, die zur Aussendung elektromagnetischer Wellen führen, die mit Antennen empfangbar und für das zu untersuchende Material charakteristisch sind. Hierbei wird durch Überlagerung von Magnetfeldern der Volumenbereich (Voxel) des untersuchten Körpers festgelegt, aus dem die empfangene elektromagnetische Welle stammt. Auch diese Meßwerte können durch Tomographie- bzw. Rückprojektionsalgorithmen zu Schichtbildern umgerechnet werden.

Auch in der Sonographie, d.h. bei Ultraschalluntersuchungen besteht die Möglichkeit, sonographische Aufnahmen zu erstellen, die auf der Reflexion der Ultraschallwellen beruhen. Weitere bekannte tomographische Vorrichtungen sind beispielsweise Digitale Volumen-Tomographen (DVT) sowie auch die folgenden Geräte: Cone Beam Computertomographen (CBCT) oder Tomographen, die zur Erfassung der Volumeninformation bei Extremitäten Verwendung finden oder Geräte zur räumlichen Darstellung der weiblichen Brust (Tomosynthese).

Für den Prüfkörper und insbesondere für das erfindungsgemäße Verfahren ist es unerheblich, welche Art von Tomograph, bevorzugt Volumentomograph eingesetzt wird, da das Verfahren die Auswertung der erstellten Schichtbilder bzw. daraus resultierender 3D-Aufnahmen betrifft.

Um aus den erstellten tomographischen Aufnahmen solcher vorgenannter Vorrichtungen eindeutige Rückschlüsse ziehen zu können auf die jeweils untersuchten Körper, wie beispielsweise die verschiedenen Gewebearten oder allgemein der Materialarten der Körper und deren Abmessungen, besteht die Notwendigkeit, dass die Übertragungseigenschaften dieser Volumentomographen Tomographen bestimmte Qualitätskriterien erfüllen.

So ist beispielsweise die so genannte Modulationsübertragungsfunktion (MTF-Modulation Transfer Function), bzw. Kontrastauflösung ein wesentliches Maß, anhand dessen eine Aussage darüber getroffen werden kann, mit welcher Güte verschiedene Gewebetypen oder allgemein Materialien eines untersuchten Körpers voneinander unterschieden werden können, wie beispielsweise gesundes Gewebe und Tumorgewebe.

Weiterhin ist es bekannt, dass Volumentomographen bzw. die eingesetzten jeweiligen, insbesondere herstellerspezifischen Algorithmen, wie die vorgenannten Rückprojektionsalgorithmen, zu geometrischen Verzerrungen führen können in der bildlichen Wiedergabe eines untersuchten Körpers gegenüber den tatsächlichen räumlichen Dimensionen des Körpers. Solche Verzerrungen bzw. zu ungenaue Ortsauflösung sind insbesondere in der Prothetik oder auch der Strahlentherapie nicht tolerierbar, da dort das Zielvolumen genauestens bestimmt werden muss, um Schädigungen von umliegendem Gewebe zu vermeiden. Die Kenntnis über solche Verzerrungen ist beispielsweise auch dann nötig, wenn bildliche Wiedergaben desselben Körpers von verschiedenen Tomographie-Systemen überlagert (gematcht) werden sollen.

Weitere beispielhafte Qualitäts-Kriterien sind das Signal-Rausch-Verhältnis oder auch das Rausch-Leistungs-Spektrum.

Im Stand der Technik ist es bekannt, Prüfkörper bereitzustellen, mit denen die Möglichkeit geschaffen wird, Volumentomographen hinsichtlich der vorgenannten Kriterien bzw. Übertragungseigenschaften, wie zum Beispiel Kontrast- und Ortsauflösung zu überprüfen. Besonders bei der radiologisch basierten Planung von Eingriffen, zum Beispiel in der Prothetik oder der Radiotherapie gilt es, die Unsicherheit der Lagebestimmung im Zielvolumen zur Schonung gesunden Gewebes möglichst klein zu halten.

Solche Prüfkörper werden daher eingesetzt, um besonders eine Überprüfung hinsichtlich Kontrast- und Ortsauflösung der Volumentomographen durchführen zu können. Es ist dabei bekannt, dass solche Prüfkörper Strukturelemente umfassen, die mittels eines Volumentomographen in der räumlichen 3D-Darstellung oder den einzelnen Schichtbildern eines Schichtbild-Stapels gegenüber der Umgebung unterschieden werden können. Solche Prüfkörper werden mit Bezug auf ihre Anwendung in der Radiologie auch als radiologische Phantome oder allgemein als Prüfkörper bezeichnet.

Ein Prüfkörper gemäß dem Oberbegriff des Anspruchs 1 ist aus der Patentschrift US-A-6076966 bekannt. Ferner offenbart der Artikel "Enhancement and Proof of Accuracy of Industrial Computed Tomography (CT) Measurements", Bartscher M. et al, 1. Januar 2007, CIRP ANNALS, ELSEVIER BV, NL, CH, FR, Bd. 56, Nr. 1, Seiten 495 - 498, drei verschiedene Prüfkörper, nämlich, einen Prüfköper mit hohlförmigen Zylindern, einen Prüfköper bestehend aus einem Stapel von Platten mit verschiedenen Durchmessern, und einen Prüfköper, der einen Stab mit Kugeln aufweist.

Es ist die Aufgabe der vorliegenden Erfindung, ein Verfahren sowie auch einen Prüfkörper zur Durchführung eines Verfahrens zum Zweck der Überprüfung der Übertragungseigenschaften von Volumentomographen bereitzustellen, mit denen die Möglichkeit besteht, mit ein und demselben Prüfkörper, insbesondere einem jeweils an den zu untersuchenden Volumentomographen angepassten Prüfkörper die Übertragungseigenschaften hinsichtlich mehrerer Kriterien gleichzeitig zu überprüfen. Beispielsweise soll die Möglichkeit geschaffen werden, die Übertragungseigenschaften hinsichtlich Kontrastauflösung und Ortsauflösung, gegebenenfalls auch hinsichtlich weiterer Kriterien, wie z.B. Signal-Rausch-Verhältnis oder Rausch-Leistungs-Spektrum zu prüfen.

Im Folgenden wird weiterhin allgemein der Begriff Tomograph verwendet, wenngleich bevorzugt ein Volumentomograph gemeint ist.

Gemäß der Erfindung wird diese Aufgabe dadurch gelöst, dass der Prüfkörper zur Überprüfung der Übertragungseigenschaften von Tomographen, insbesondere von radiologischen Tomographen, mehrere zu einem Stapel verbundene Platten aufweist, in welchem benachbarte Platten einander kontaktieren und wobei der Prüfkörper Kugeln aus wenigstens einem zu den Platten verschiedenen Material von wenigstens zwei verschiedenen Durchmessern aufweist, wobei die jeweiligen Kugeln in oder zwischen wenigstens einigen der Platten angeordnet sind und wobei am/im Prüfkörper, bevorzugt in dem Stapel wenigstens ein stabförmiges Hohlprofil angeordnet ist. Ein solches Hohlprofil kann bevorzugt ein Kreisprofil im Querschnitt senkrecht zu seiner Längserstreckung aufweisen und somit ein rundes Rohr ausbilden.

Es kann hierbei in einer Ausführung vorgesehen sein, dass die Platten durch äußere oder innere Verbindungselemente zu dem Stapel verbunden sind, welche auch durch wenigstens ein Hohlprofil gebildet werden können. Die Platten können auch z.B. verklebt sein. Eine mögliche Ausführung kann es auch vorsehen, dass die Platten zu dem Stapel mittels eines vorgenannten, insbesondere eines einzigen stabförmigen Hohlprofils verbunden sind, beispielsweise welches die Platten durchdringt.

Sofern der Plattenstapel wenigstens ein stabförmiges Hohlprofil in seinem Inneren aufweist, kann es bevorzugt vorgesehen sein, dass das wenigstens eine Hohlprofil so angeordnet ist, dass es wenigstens einen Teil der Platten, ggfs. auch alle Platten durchdringt. Z.B. kann das wenigstens eine Hohlprofil hierbei mit seiner Längsachse senkrecht orientiert sein zu den Plattenebenen bzw. deren Kontaktebene. Auch eine Winkelabweichung im Bereich von plus/minus 30° von der senkrechten Orientierung kann bevorzugt vorgesehen sein, wie später noch erläutert wird.

Ein solcher Prüfkörper kann in einem Verfahren zur Überprüfung der Übertragungseigenschaften gemäß der Erfindung eingesetzt werden, um mittels eines zu prüfenden Tomographen eine Serie mehrerer Messwertaufnahmen von dem Prüfkörper zu erstellen und sodann aus dieser Serie mittels eines Tomographie-Algorithmus im Tomographen oder in einer separaten Auswerteeinheit des Tomographen einen Stapel mehrerer Schichtbilder des Prüfkörpers zu berechnen.

Bei dem erfindungsgemäßen Verfahren ist es vorgesehen, dass für wenigstens einen Teil, bevorzugt alle Schichtbilder des erstellten Bildstapels anhand der Bildwiedergabe der Querschnitte des wenigstens einen in dem Stapel angeordneten stabförmigen Hohlprofils ein Maß für die Modulation-Übertragungs-Funktion berechnet wird.

Sofern der Prüfkörper bei der Überprüfung eines Tomographen derart positioniert ist, dass in der späteren bildlichen Wiedergabe das wenigstens eine stabförmige Hohlprofil mit seiner Längsachse senkrecht zur Ebene der einzelnen Schnittbilder liegt, weist die Querschnittsdarstellung dieses Hohlprofils eine Ringform, insbesondere eine Kreisringform auf, so dass die Überprüfung der Übertragungseigenschaften des Tomographen anhand dieses wenigstens einen Hohlprofils innerhalb von jedem Schnittbild des Bilderstapels vorgenommen werden kann. So kann mit einer solchen Konstruktion in jedem Schnittbild die Prüfung anhand exakt gleicher Kriterien erfolgen.

Es kann hier bei solchen Tomographen, welche eine Strahlungsquelle auf einer Kreisbahn um eine Rotationsachse führen, auf welcher der Kreismittelpunkt der Rotation liegt, vorgesehen sein, einen Prüfkörper so zu positionieren, dass dessen Längsachse zumindest parallel zur Rotationsachse des Tomographen liegt, insbesondere so, dass die Rotationsachse im Hohlprofil liegt, besonders bevorzugt so, dass die Mittellängsachse des wenigstens einen Hohlprofils und die Rotationsachse aufeinander liegen.

Es kann auch allgemein vorgesehen sein, den Prüfkörper so zu positionieren, dass das Isozentrum des Tomographen im Inneren des wenigstens einen Hohlprofils liegt, bevorzugt auf dessen Mittellängsachse.

Sofern der Prüfkörper derart positioniert wird, dass die Längsachse nicht exakt senkrecht zur Ebene der Schnittbilder liegt, ergibt sich in der bildlichen Darstellung eine Elliptizität, aufgrund der auf die Winkelabweichung zwischen Längsachse und Schnittbildebene geschlossen werden kann.

Es ist ein weiterer wesentlicher Kerngedanke der Erfindung, dass der Prüfkörper in dessen Volumen, welches im Wesentlichen von den gestapelten Platten gebildet wird, Kugeln unterschiedlicher Durchmesser aufweist. Der Prüfkörper weist wenigstens eine Gruppe von Kugel mit einem ersten Durchmesser und eine Gruppe von Kugel mit einem zweiten Durchmesser auf.

Es kann sodann erfindungsgemäß vorgesehen sein, diese Kugeln unterschiedlicher Durchmesser zum Zweck der Überprüfung unterschiedlicher Übertragungseigenschaften zu nutzen, z.B. einerseits zur Prüfung der MTF mit den größeren / größten Kugeln und der geometrischen Verzerrungen mit kleineren als den größten Kugeln.

Es besteht so die Möglichkeit, einen Prüfkörper der erfindungsgemäßen Art zur Prüfung verschiedener Eigenschaften des Tomographen einzusetzen, ohne dass der Prüfkörper geändert oder gewechselt werden muss.

Die Anordnung von Kugeln zusätzlich zu dem wenigstens einen stabförmigen Hohlprofil hat den Vorteil, dass unabhängig von einer evtl. Winkelabweichung zwischen der Längsachse des wenigstens einen Hohlprofils und den Schnittebenen die Schnittbilder von Kugel immer Kreisscheibenform aufweisen, so dass die MTF auch anhand dieser Kugel bestimmt werden kann, insbesondere anhand einiger der Kugeln (gleichen Durchmessers), insbesondere der Kugeln mit dem größten Durchmesser.

Eine Weiterbildung kann auch vorsehen, dass anhand der Bildwiedergabe der Querschnitte von Kugeln mit anderem Durchmesser, insbesondere der Kugeln mit einem kleineren als den größten Durchmesser entweder die jeweilige Abweichung einer Kugel in der Bildwiedergabe von ihrer bekannten Soll-Position berechnet wird oder die Abweichungen der Kugeln in der Bildwiedergabe untereinander von ihren bekannten Soll-Abständen berechnet werden.

Erfindungsgemäß kann es dabei vorgesehen sein, die Materialien der in dem Prüfkörper eingesetzten Komponenten, also beispielsweise der Platten, des wenigstens einen stabförmigen Hohlprofils und der Kugeln auszuwählen in Abhängigkeit des Mess-Prinzips, welches dem zu prüfenden Tomographen zu Grunde liegt.

Mit beispielhaften Bezug auf einen Tomographen, der mit Röntgenstrahlung arbeitet, kann es demnach vorgesehen sein, die Platten aus einem Material zu wählen, welches Absorptionseigenschaften für Röntgenstrahlung aufweist, die denen von Wasser gleichkommen oder zumindest ähneln. Ein solches Material ist im Stand der Technik grundsätzlich bekannt und wird auch als "Solid-Water" bezeichnet. So können zur Simulation von Wasser beispielsweise bestimmte Kunststoffe eingesetzt werden, wie Mischungen unterschiedlicher Kunststoffe, wie Polyethylen oder Polystyrol mit Titandioxid, die so aufeinander abgestimmt werden, dass dieselbe Elektronendichte entsteht, die Wasser besitzt.

Es kann demnach vorgesehen sein, zumindest einige der Platten, ggfs. auch alle Platten eines Prüfkörpers gemäß der Erfindung aus einem solchen Material zu wählen, welches Absorptionseigenschaften wie Wasser aufweist, wobei Kugeln der unterschiedlichen Durchmesser sowie gegebenenfalls auch das wenigstens eine stabförmige Hohlprofil, welches die Platten beispielsweise senkrecht zur Plattenebene oder in einem davon abweichenden Winkel durchdringt, aus Metall, beispielsweise aus Aluminium oder auch Magnesium gefertigt sind. Metalle, insbesondere Aluminium oder Magnesium sind dafür bekannt, Absorptionseigenschaften für Röntgenstrahlung aufzuweisen, die denen von Knochen ähneln. Statt wenigstens einem die Platten innen zumindest teilweise durchdringenden Element, wie wenigstens einem stabförmigen Hohlprofil kann es auch vorgesehen sein, wenigstens ein äußeres stabförmiges Hohlprofil um die Platten herum anzuordnen, z.B. als äußere Stäbe oder als äußeren Käfig vorzusehen, insbesondere mit dem/denen die Platten verbunden werden können.

Auch hier kann es sodann vorgesehen sein, einen Prüfkörper mit den ggfs. mehreren Hohlprofilen, die nicht auf derselben Achse, jedoch bevorzugt parallel zueinander liegen, so in einem Tomographen zur Überprüfung zu orientieren, dass die Hohlprofile senkrecht zu den Schnittbildebenen liegen, damit die Schnittbilder der Hohlprofile Kreisringform aufweisen.

Besonders die hohle Ausbildung des stabförmigen Hohlprofils hat gegenüber massiven Stäben den Vorteil, weniger Artefakte in den bildlichen Darstellungen aufzuweisen.

Hier kann es als nicht beschränkendes Beispiel vorgesehen sein, dass der äußere Durchmesser des Hohlprofil gewählt wird zu 14mm und die Wandstärke zu 4 mm. Die Parameter von äußeren Durchmesser und Wandstärke können bevorzugt in Abhängigkeit von der Strahlqualität gewählt werden.

Durch die vorgenannten Materialien kann demnach ein Prüfkörper passend für einen Röntgen-Tomographen ausgewählt werden.

Sollen hingegen Tomographen überprüft werden, die auf dem Magnetresonanzprinzip beruhen, kann es auch vorgesehen sein, Platten und Kugeln, sowie das wenigstens eine stabförmige Hohlprofil jeweils aus nichtmetallischen, zumindest aber aus nicht magnetisierbaren / nicht magnetischen Materialien auszuwählen, wobei die Kugeln sowie die Platten zwecks Erzielung einer Unterscheidbarkeit in der bildlichen Wiedergabe des Tomographen aus verschiedenen Materialien ausgewählt sein können, insbesondere mit verschiedenen Protonendichten.

Erfindungsgemäß werden in dem Prüfkörper Kugeln eingesetzt, da diese symmetrisch sind und unabhängig von der Lage der Schnittebene in der bildlichen Wiedergabe der jeweiligen Schichtbilder eines Bilderstapel immer einen Kreisflächenquerschnitt darstellen, der auf vergleichsweise einfache Art und Weise weiter auswertbar ist.

Neben den vorgenannten Kugeln von wenigstens zwei verschiedenen Durchmessern kann es vorgesehen sein, dass Kugeln mit einem kleinsten Durchmesser im Prüfkörper angeordnet sind, die als so genannte Ankerpunkte dienen, um die bildliche Wiedergabe des Prüfkörpers hinsichtlich seiner Lage in dem der Auswertung zu Grunde liegenden Koordinatensystem zu korrigieren, was möglich ist, wenn die Lage der als Ankerpunkte dienenden Kugeln im Prüfkörper ebenso wie die Lage der weiteren Kugeln mit hinreichender Genauigkeit bekannt ist. Es kann beispielsweise vorgesehen sein, die Lage der jeweils in die Platten integrierten Kugeln mit einer Genauigkeit von besser als 0,1 mm vorzugeben.

In einer Ausführung kann es vorgesehen sein, dass Kugel mit genau drei verschiedenen Durchmessern im Prüfkörper eingesetzt werden. Hier dienen die kleinsten Kugeln als Ankerpunkte, insbesondere um die Lage des Prüfkörpers für die Auswertung rechnerisch zu korrigieren, die Kugeln mit mittleren Durchmesser dazu, die geometrischen Verzerrungen zwischen bildlicher Darstellung und der tatsächlichen Geometrie zu prüfen und die größten Kugeln dazu, ein Maß für die Modulations-Übertragungsfunktion zu bestimmen.

In einer vorteilhaften Ausführungsform des erfindungsgemäßen Prüfkörpers kann es weiterhin vorgesehen sein, dass dessen äußere Form zumindest im Wesentlichen einem menschlichen oder tierischen Körperteil nachempfunden ist. So kann zum Beispiel ein Prüfkörper derart ausgebildet sein, dass die gestapelten und mit dem Element verbundenen Platten im Wesentlichen eine Kugelform ausbilden, umso zum Beispiel den Kopf eines Menschen zu simulieren. Hier kann es beispielsweise vorgesehen sein, dass die Durchmesser der einzelnen gestapelten in diesem Fall kreisscheibenförmigen Platten ausgehend von einer mittig angeordneten Platte mit größtem Durchmesser in Richtung nach außen, ggfs. in Stufen, abnehmen.

In einer anderen Ausführungsform kann es auch vorgesehen sein, Platten mit gleichem Kreisquerschnitt d.h. mit gleichem Durchmesser oder mit nur in einer Richtung sich ändernden Kreisquerschnitt (Durchmesser) zu stapeln und so einen im Wesentlichen zylindrischen oder sich in einer Richtung verjüngenden Prüfkörper zu formen, welcher im Wesentlichen einer Extremität des Menschen oder eines Tieres ähnelt, wie beispielsweise einem Arm oder einem Bein.

In einer wiederum anderen Ausführungsform kann es auch vorgesehen sein, die einzelnen Platten derart zu formen, dass der Stapel die Form einer weiblichen Brust erhält, was insbesondere dann vorteilhaft sein kann, wenn tomographische Vorrichtungen zur Durchführung einer Mammographie überprüft werden sollen.

Es kann somit ein Tomograph vor der Durchführung einer eigentlichen Untersuchung oder auch einer medizinischen Anwendung mit einem je nach Anwendungsfall ausgewählten Prüfkörper hinsichtlich seiner Übertragungseigenschaften geprüft werden.

Besonders bei rotationssymmetrischen Prüfkörpern, z.B. zylindrischen kann es vorgesehen sein, dass wenigstens ein stabförmiges Hohlprofil in bzw. auf dessen geometrischer Mittenachse angeordnet wird, dass also die Mittenlängsachsen des wenigstens einen Hohlprofil und des Prüfkörpers zusammenfallen.

In einer bevorzugten Ausführungsform des Prüfkörpers kann es weiterhin vorgesehen seien, dass die Kugeln jeweils angeordnet sind in einer Ausnehmung in der Oberfläche einer Platte, welche entweder durch die plane Oberfläche wenigstens einer benachbarten Platte oder eine gegenüberliegende weiterer Ausnehmung in einer benachbarten Platte überdeckt ist. Auf diese Art und Weise besteht eine konstruktiv einfache Möglichkeit die Kugeln in das Volumen der Platten bzw. des für die Platten verwendeten Materials zu integrieren.

Im einfachsten Fall kann eine jeweilige Kugel vollständig d.h. mit ihrem gesamten Volumen in einer Ausnehmung in einer Platte aufgenommen sein, wofür diese Platte zum Beispiel eine zylindrische Bohrung, ggfs. eine vollständige Durchgangsbohrung aufweist. Diese Bohrung kann sodann zum Beispiel durch die Fläche einer oder auch zweier (von beiden Seiten) benachbarten kontaktierenden Platte überdeckt sein, so dass die Lage einer in der Bohrung positionierten Kugel fixiert ist.

In anderer Ausführung besteht auch die Möglichkeit in die Oberfläche einer Platte eine halbkugelförmige Ausnehmung einzubringen, die von der kontaktierenden Fläche einer benachbarten Platte mit einer ebenfalls halbkugelförmigen Ausnehmung überdeckt ist, wobei die beiden sich überdeckenden Ausnehmungen sich insgesamt zu einem exakt kugelförmigen Volumen ergänzen mit dem Durchmesser einer aufzunehmenden Kugel, so dass in einem solchen Fall eine zwischen zwei Platten mit der Kugelmitte exakt in der Kontaktebene der Platten aufgenommene Kugel ohne jeglichen Lufteinschluss aufgenommen ist.

In einer einfacheren Ausgestaltung kann auch vorgesehen sein, dass die jeweiligen Ausnehmungen nicht an die Kugelform angepasst sind, sondern jeweils einander gegenüberliegende zylindrische Ausnehmungen / Bohrungen bilden.

Besonders zum Zweck der Durchführung einer späteren Auswertung in Verbindung mit dem erfindungsgemäßen Verfahren kann es in einer Weiterbildung vorgesehen sein, dass einige der Kugeln, insbesondere diejenigen Kugeln mit dem größten Durchmesser in Abhängigkeit der Größe eines Pixels oder Voxels (der Flächeneinheit oder der Volumeneinheit in/aus der die Meßwerte erfasst werden. bzw. das zu erfassende Signal stammt) der Aufnahmesensorik eines zu prüfenden Tomographen derart ausgewählt sind, dass deren Durchmesser dem 20- bis 70-fachen dieser Größe entspricht.

Es kann also vorgesehen sein, dass zunächst festzulegen ist, welche Art von Tomograph zu überprüfen ist und anhand der Größe der Bild- oder Volumenauflösung der Aufnahmesensorik dieses Tomographen, beispielsweise eines auf Röntgenstrahlung basierenden Computertomographen oder eines MRT sodann die Kugelgröße bzw. der einzusetzende Prüfkörper mit entsprechend passenden Kugelgrößen ausgewählt wird.

Hierfür besteht beispielsweise ganz allgemein die Möglichkeit, einen Satz von mehreren Prüfkörpern gleicher oder auch unterschiedlicher Form bereitzustellen, die neben der Möglichkeit verschiedene Körperteile zu simulieren auch hinsichtlich der Kugelgrößen an die Auflösung (bildliche Flächen- oder Volumenauflösung) der zu prüfenden Tomographen angepasst sind und somit ein Benutzer zum Zweck der Überprüfung eines Tomographen aus einem solchen Satz von verschiedenen Prüfkörpern den für Überprüfung geeigneten Prüfkörper auswählen kann. In einer möglichen Ausführung kann es hier beispielsweise vorgesehen sein, dass der Kugeldurchmesser dieser Kugeln mit dem größten Durchmesser innerhalb des Prüfkörpers im Bereich von 5-10 mm liegt.

In einer weiteren, insbesondere mit der vorherigen kombinierbaren Ausführung kann es vorgesehen sein, dass einige, insbesondere alle Kugeln mit einem kleineren als dem größten Durchmesser in Abhängigkeit der Größe eines Pixels oder Voxels der Aufnahmesensorik (z.B. Bildsensor oder Volumenelement, aus dem das Signal stammt) eines zu prüfenden Tomographen derart gewählt sind, dass deren Durchmesser dem 10- bis 20-fachen dieser Größe entspricht. Hier ist es insbesondere vorgesehen, mit diesen Kugeln, die kleiner sind als der maximale Kugeldurchmesser aller vorkommenden Kugeln, die dreidimensionale Abbildungsgenauigkeit, d.h. geometrische Verzerrungen des Tomographen überprüfen zu können.

Zu diesem Zwecke ist es vorteilhaft, wenn diese Kugeln einen kleineren als den größten Durchmesser aufweisen. Ein üblicher Kugeldurchmesser kann hier im Bereich bei diesen kleineren Kugeln von 1 bis 5 mm liegen.

Die Kugeln mit dem größten Durchmesser hingegen können zur Erfassung eines Maßes der Modulations-Übertragungs-Funktion eingesetzt werden.

In einer Weiterbildung, die mit allen vorher genannten Ausführungsformen kombinierbar ist, kann es vorgesehen sein, dass die einzelnen einander kontaktierenden Platten von einem die Platten verbindenden, z.B. durchdringenden Element zusammengehalten werden, welches beispielsweise stabförmig Ringquerschnitt ausgebildet ist und die einzelnen Platten exakt senkrecht zur jeweiligen Plattenober- bzw. Unterfläche durchdringt und ein eingangs genanntes Hohlprofil ausbildet.

Eine andere Ausführung kann vorsehen, dass in dem Stapel der Platten zwar wenigstens ein stabförmiges Hohlprofil angeordnet ist, dieses jedoch keine Verbindungsfunktion ausübt. Z.B. kann das wenigstens eine Hohlprofil, insbesondere Rohr in einem Kanal angeordnet sein, der im Stapel zumindest teilweise ausgebildet ist, d.h. zumindest durch einige der den Stapel bildenden Platten verläuft, ggfs. den Stapel komplett durchdringt. Hierfür kann jede oder zumindest einige der Platten eine Durchgangsbohrung aufweisen. Zur Bildung des wenigstens einen Kanals sind die Durchgangsbohrungen der Platten im Stapel fluchtend angeordnet.

Ein Prüfkörper kann bei seiner bestimmungsgemäßen Verwendung bei der Überprüfung eines Tomographen zum Beispiel derart positioniert werden, dass das die Platten verbindende Element, insbesondere der Stab senkrecht zu den Schichtebenen der späteren Schichtbilder des Bilderstapels orientiert ist.

Dies hat den eingangs genannten Vorteil, dass die bildliche Wiedergabe des Stabes in jedem der Schichtbilder einem Ringquerschnitt entspricht. Bei dieser Ausführungsform, sowie der vorgenannten Ausführung, die Kugeln im Volumen des Körpers zu positionieren, können diese Kugeln lediglich in diskreten Bereichern innerhalb des Volumens des Prüfkörpers positioniert werden.

Diese diskreten Bereiche sind abhängig von der Art der Positionierung der Kugeln im Prüfkörper und z.B. durch die Kontaktebene zweier benachbarter Platten gegeben oder durch die jeweiligen Oberflächenbereiche einer jeweiligen Platte, in welche eine jeweilige Kugel komplett oder zumindest zum Teil eingelassen ist.

So kann es beispielsweise auch vorgesehen sein, dass eine Platte, welche Kugeln aufnimmt, Durchgangsbohrungen senkrecht zur Plattenoberfläche aufweist und somit das Volumen zur Aufnahme einer Kugel durch die gesamte Dicke der Platte gegeben ist, welche sodann von zwei benachbarten Platten zum Abschluss dieses Volumens und zur Aufnahme der Kugeln kontaktiert wird. In einem solchen Fall können Kugeln jeweils nur diskret mit ihrem Mittelpunkt in der Mitte einer jeweiligen aufnehmenden Platte positioniert sein.

Um die Möglichkeit zu schaffen, Kugeln innerhalb des Prüfkörpers nicht lediglich in diskreten Positionen anzuordnen, kann in einer Weiterbildung vorgesehen sein, dass die Kontaktebenen, in denen die einander kontaktieren Oberflächen benachbarter Platten liegen, zum Beispiel abweichend von einer senkrechten Anordnung relativ zum stabförmigen Hohlprofil liegen. Z.B. kann ein Winkelbereich von plus/minus 30° um die senkrechte Anordnung gewählt werden.

Wird ein Prüfkörper dieser Ausgestaltung derart zur Überprüfung eines Tomographen in diesem positioniert, dass das stabförmige Hohlprofil in der bildlichen Wiedergabe senkrecht zu den Schichtebenen des Bildstapels verläuft, so liegen die Kontaktebenen der einzelnen Platten nicht mehr parallel zu den einzelnen Schichten des Bilderstapels, sondern in einem (von 0 bzw. 180 Grad verschiedenen) Winkel hierzu, so dass die Kugeln die in oder zwischen benachbarten Platten angeordnet sind bezogen auf die einzelnen Schichtbilder verschiedene Höhenlagen einnehmen können.

Beispielsweise sofern ein Prüfkörper mit wenigstens einem stabförmigen Hohlprofil nicht zwingend derart in einem zu prüfenden Tomographen angeordnet wird, dass das Hohlprofil in der späteren Schichtbildfolge senkrecht zu der Schichtbildebene verläuft, kann es mit allgemeiner Gültigkeit vorgesehen sein, dass bei einer bestimmungsgemäßen Anordnung des Prüfkörpers in einem zu prüfenden Tomographen die Kontaktebenen der einander kontaktieren Oberflächen benachbarter Platten nicht parallel zu den Ebenen der von dem Tomographen oder einer externen Auswerteeinheit berechneten Schnittbilder eines Schnittbilderstapel liegen, bzw. gelegt werden, um diesen vorgenannten Vorteil zu erzielen.

Es besteht demnach durch eine der beiden vorgenannten Ausführungsformen die Möglichkeit zu realisieren, dass in einem jeweiligen Schichtbild die bildlichen Wiedergaben der Kugeln unterschiedliche Querschnittgrößen aufweisen, selbst wenn die in der bildlichen Wiedergabe vorhandenen Kugeln tatsächlich alle den gleichen Durchmesser haben, da mit der vorgenannten Ausführungsform diese Kugeln in den jeweiligen Schichtbildern in verschiedenen Höhenlagen geschnitten sind.

Ein Verfahren zur Überprüfung der Übertragungseigenschaften eines Tomographen benutzt bevorzugter Weise einen Prüfkörper gemäß der Erfindung, insbesondere der zuvor beschriebenen Art.

Hier kann es in einer Ausführungsform vorgesehen sein, dass für die Berechnung eines Maßes der Modulation-Übertragungs-Funktion (MTF) in wenigstens einem der Schichtbilder, bevorzugt in allen Schichtbildern durch eine Bilderkennungssoftware oder aber auch durch manuelle Auswahl von einem Bediener wenigstens eine Bildwiedergabe des Querschnitts einer Kugel oder des Querschnitts des wenigstens einen stabförmigen Hohlprofils selektiert wird, um sodann dieses so selektierte bzw. ausgewählte Querschnitts-Bild der Kugel oder des Hohlprofils hinsichtlich des Maßes der Modulation-Übertragungs-Funktion auszuwerten.

Grundsätzlich unabhängig davon, mit welcher Art und Weise die bildliche Wiedergabe einer Kugel oder des Hohlprofils innerhalb eines Schnittbildes identifiziert wird, kann es für die Berechnung eines Maßes der Modulations-Übertragungs-Funktion vorgesehen sein, dass alle Intensitäts-Messwerte der eine bildliche Wiedergabe wenigstens eines Querschnitts einer Kugel, insbesondere einer solchen mit dem größten Durchmesser oder wenigstens eines Hohlprofils repräsentierenden Pixel oder Voxel gemittelt werden, die innerhalb eines vorgegebenen Toleranzintervalls den gleichen radialen Abstand zum Mittelpunkt der bildlichen Wiedergabe haben. Hierfür kann es z.B. vorgesehen sein, diesen Mittelpunkt durch eine Bildauswertung automatisch zu bestimmen.

Hierbei bedeutet das Durchführen einer Mittelung, dass die Intensitäts-Messwerte dieser Pixel/Voxel mit jeweils gleichem radialen Abstand summiert werden und das Ergebnis dieser Summe durch die Anzahl der summierten Pixel / Voxel geteilt wird. Durch diese Verfahrensvariante kann eine Art der Überabtastung durchgeführt werden, da der Abstand von Radien geringer wird, als das einzelne Detektorelement bzw. Volumenelement aus dem das Signal stammt (Sensor-Pixel bzw. Voxel).

Es besteht sodann in einer vorteilhaften Weiterbildung die Möglichkeit, dass für die funktionale Abhängigkeit der gemittelten Intensitäts-Messwerte vom Radius eine Funktion, beispielsweise ein Polynom gefittet bzw. gebildet wird. Diese gebildete / gefittete Funktion stellt sodann im Sinne der Erfindung ein Maß dar, mit dessen Hilfe die Modulations-Übertragungs-Funktion (MTF) ermittelt werden kann. Die MTF kann verwendet werden, um die Qualität des Tomographen zu prüfen.

Wie eingangs erwähnt werden können diejenigen Kugeln, die einen kleineren Durchmesser haben als die Kugeln mit dem größten Durchmesser innerhalb des Prüfkörpers bevorzugt eingesetzt werden, um den Tomographen bzw. dessen Auswerte- Software (Rückprojektionsalgorithmus) zu überprüfen hinsichtlich geometrischer Verzerrungen d.h. hinsichtlich der 3-D-Abbildungsgenauigkeit.

So ist die jeweilige Position dieser Kugeln innerhalb des Prüfkörpers bekannt, beispielsweise relativ zum Mittelpunkt des Prüfkörpers, welcher in einer beispielhaften Ausführung axial in der Mitte des wenigstens einen Hohlprofils liegen kann.

In Kenntnis dieser bekannten Positionen der Kugeln besteht sodann die Möglichkeit, innerhalb der jeweiligen bildlichen Wiedergaben d.h. der Darstellung der Kugeln in den einzelnen Schichtbildern bzw. einer aus den Schichtbildern zusammengesetzten 3-D Darstellung die Abweichung entweder der jeweiligen Kugeln von der Sollposition oder die relative Abweichung von Kugeln untereinander zu bestimmen und für jede der nach diesem Kriterium untersuchten Kugeln die Größe der Abweichung im Schichtbild oder in der gebildeten 3-D Darstellung zu visualisieren.

Um eine solche Visualisierung durchzuführen kann es in einer Ausführungsform beispielsweise vorgesehen sein, dass die Kugelwiedergaben in den Schichtbildern bzw. der 3D-Darstellung farbig markiert werden, wobei die Farben von der Größe der Abweichung abhängen. Auch können die Abweichungen als Zahlenwert wiedergegeben werden, z.B. prozentual. Hierdurch sind für einen Betrachter die Abweichungen von den Sollpositionen bzw. die geometrischen Verzerrungen optisch gut erkennbar. Solche Ergebnisse können z.B. verwendet werden, um die Auswertealgorithmen bzw. Rückprojektionsalgorithmen zu optimieren oder auch ein Matching der Bilder verschiedener Tomographen zu ermöglichen.

Eine mit jeder der zuvor genannten Ausführungsformen kombinierbare Weiterbildung kann es vorsehen, dass in wenigstens einem der stabförmigen Hohlprofile, ggfs. in dem einzigen Hohlprofil wenigstens eine Sensorvorrichtung angeordnet ist. Eine solche Sensorvorrichtung kann z.B. eingesetzt werden, um die Dosis der bei einer Überprüfung aufgetretenen Strahlung zu messen. Da durch die zumindest teilweise Abschirmung von Strahlung, z.B. Röntgenstrahlung durch das Material des Hohlprofiles eine systematische Fehlmessung der tatsächlichen Dosis stattfindet, kann es vorgesehen sein, die Messwerte nach der Erfassung zu korrigieren, z.B. anhand der bekannten tatsächlichen Abschirmung.

Um Abschirmungseffekte zu vermeiden kann eine demgegenüber bevorzugte Ausführungsform vorsehen, dass in dem Stapel von Platten wenigstens ein Kanal angeordnet ist, der zumindest einige der Platte durchdringt. Neben der Möglichkeit, Hohlprofile aus verschiedenen Materialien je nach Anwendungsfall in den Kanal einzuschieben, besteht hier auch die Möglichkeit in dem wenigstens einen Kanal wenigstens zwei in axialer Richtung beabstandete Hohlprofile, insbesondere Rohre mit Kreisringquerschnitt anzuordnen.

Es wird so ein Abstandsbereich zwischen zwei solchen Hohlprofilen im Inneren des Plattenstapel gebildet, bevorzugt in dessen Mitte, der nicht von dem Material der Hohlprofile umgeben ist, insbesondere also frei von abschirmendem Metall ist. Es kann bei dieser Ausführungsform vorgesehen sein, wenigstens eine Sensorvorrichtung, z.B. zur Erfassung der Strahlendosis bei der Überprüfung in einem solchen Abstandsbereich anzuordnen, z.B. indem wenigstens eine solche Sensorvorrichtung durch eines der Hohlprofile oder auch ggfs. mehrere in den Abstandsbereich geschoben wird.

Eine solche Sensorvorrichtung kann z.B. durch sogenannte Solid-State-Detektoren, Dioden, Thermolumineszenz-Kristalle oder ähnliches ausgebildet sein.

Z.B. können so unterschiedliche Mess-Serien anhand des parallel erfassten Dosis-Messwertes normiert werden bzw. miteinander verglichen werden. Auch besteht die Möglichkeit die Überprüfung eines Tomographen in Abhängigkeit der Dosis vorzunehmen und so von der Dosis abhängige Prüfergebnisse, insbesondere MTF oder geometrische Verzerrungen zu bestimmen.

Beispielsweise kann für eine evtl. später folgende Patientenuntersuchung eine minimale Strahlen-Dosis bestimmt werden um qualitativ einwandfreie Abbildungen zu erhalten, wodurch Patienten geschont werden. Anhand von Dosis-abhängigen Prüf-Messwerten kann demnach eine Dosis so hoch wie nötig und so gering wie möglich eingestellt werden.

Die Ausführung eines Prüfkörpers mit Plattenstapel und wenigstens einem Kanal mit wenigstens einem darin angeordneten Hohlprofil hat weiterhin den Vorteil, dass eine Haltevorrichtung zum Festhalten eines Prüfkörpers in einem zu überprüfenden Tomographen zumindest teilweise in das Innere wenigstens eines Hohlprofils eingreifen kann, ggfs. sogar komplett durch den Prüfkörper hindurchgreifen kann. Z.B. kann ein Prüfkörper mit durch alle Platte hindurchreichendem wenigstens einen Hohlprofil auf einem Haltestab oder Halteband aufgefädelt werden. Auch kann ein Laserstrahl durch das Innere des wenigstens einen Hohlprofil hindurchgeführt werden, um einen Prüfkörper z.B. auf ein Isozentrum eines Tomographen zu justieren.

Eine weitere mit jeder der zuvor genannten Ausführungsformen kombinierbare Weiterbildung kann es vorsehen, dass in dem Plattenstapel zusätzlich zu wenigstens einem stabförmigen Hohlprofil oder wenigstens zwei stabförmigen mit axialem Abstand hintereinander liegenden Hohlprofilen, mehrere Stäbe, insbesondere aus einem Vollmaterial vorgesehen sind, die um wenigstens eines der Hohlprofile herum angeordnet sind. Hierbei kann jeder der Stäbe parallel oder bevorzugt in einem von 0° abweichenden Winkel angeordnet sein, z.B. in einem Winkel von bis zu 10 Grad. Beispielsweise kann jeder der zusätzlichen Stäbe auf dasjenige Hohlprofil zulaufen, um das er herum angeordnet ist.

Eine bevorzugte Ausführung kann dabei vorsehen, dass die zusätzlichen Stäbe nur einen Teil der Platten aller Platten durchdringen. Z.B. können die zusätzlichen Stäbe ausgehend von einer letzten/ersten Platte des Plattenstapels sich in den Stapel hinein erstrecken, z.B. nur über einen Bereich von nicht mehr als 50% der gesamten Stapellänge (gemessen in einer Richtung senkrecht zu den Kontaktebenen der Platten), bevorzugt nicht mehr als 30% der gesamten Stapellänge. Die zusätzlichen Stäbe sind bevorzugt im Durchmesser kleiner als das wenigstens eine Hohlprofil, insbesondere welches sie umgeben.

Ein Prüfkörper mit einer der vorgenannten Ausführungen der zusätzlichen Stäbe kann eingesetzt werden, um an diesem wenigstens ein erstes Bild oder eine ersten Bildreihe aufzunehmen und wenigstens ein zweites Bild oder eine zweite Bildreihe, wobei die ersten und zweiten Bilder oder Bildreihen in axialer Richtung des Hohlprofils, das von den Stäben umgeben ist, versetzt sind. Es besteht so die Möglichkeit die ersten und zweiten Bilder oder Bildreihen zusammenzufügen und so zu überprüfen, ob in dem zusammengefügten Bild oder der zusammengefügten Bildreihe ein lateraler und/oder axialer Versatz bei den jeweiligen zusätzlichen Stäben vorliegt oder nicht vorliegt. Der Vorgang des sogenannten "Stitching" oder Zusammenfügen von Bildern kann hierdurch überprüft werden. Ein solcher Vorgang ist z.B. nötig, wenn mit einem Tomographen separate Aufnahmen von Ober- und Unterkiefer erstellt werden, die nachträglich zu einer Gesamtdarstellung zusammengefügt werden sollen. Hierfür ist es wichtig zu wissen, dass der Vorgang des "Stitching" und die Erstellung der separaten Bilder / Bildreihen korrekt vorgenommen wird.

Mit einem Prüfkörper und einem Verfahren der erfindungsgemäßen Art kann erkennbar mittels einer einzigen Aufnahmeserie der jeweils untersuchte Tomograph hinsichtlich seiner Übertragungsleistungen anhand von mehreren Kriterien gleichzeitig überprüft werden. Ein und derselbe Bilderstapel, umfassend mehrere Schichtbilder, kann gleichzeitig oder nacheinander mit einer Auswertesoftware hinsichtlich dieser Kriterien untersucht werden, um so jeweils ein Maß für das untersuchte Kriterium zu erhalten.

Ausführungsformen der Erfindung werden anhand der nachfolgenden Figuren näher erläutert:

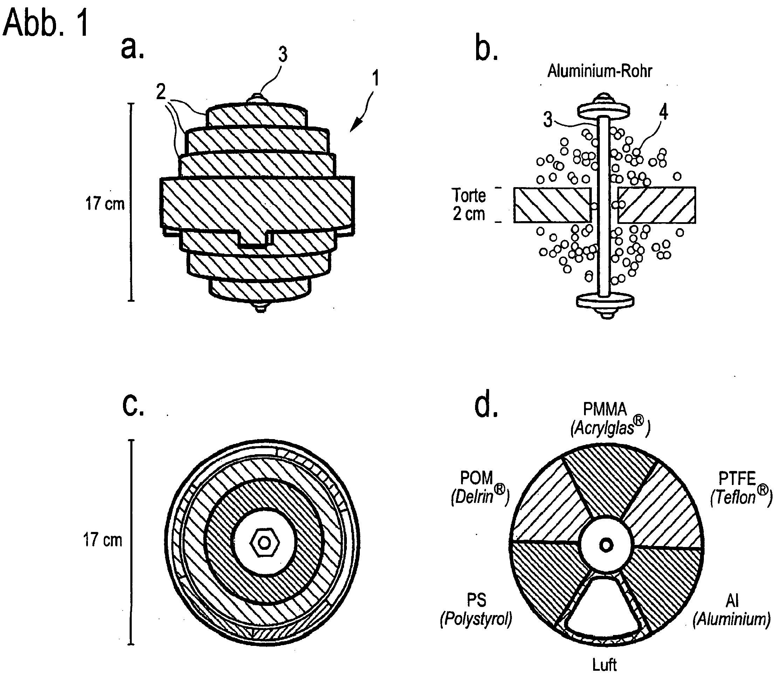

Die Abbildung 1 zeigt in einer seitlichen Ansicht und in Aufsicht einen Prüfkörper 1 gemäß der Erfindung, der zusammengesetzt ist aus mehreren übereinander gestapelten Platten 2, die hier durch ein stabförmiges Rohr 3, welches die Platten 2 jeweils mittig durchdringt, zu einem Stapel verbunden sind. Hierbei ist in dieser Ausführung das stabförmige Hohlprofil 3 aus einem Aluminium-Rohr gebildet, welches Gewinde an seinen Enden hat, um so mit Muttern auf diesen Gewindeenden die Platten 2 aneinander zu drücken.

Wie im allgmeinen Teil beschrieben, ist die Erfindung nicht auf eine solche Konstruktion beschränkt. Es kann auch vorgesehen sein, im Stapel oder außen an diesem wenigstens ein Rohr anzuordnen, dass keine die Platten 2 verbindende Funktion ausübt.

In Oberflächenbereichen der einzelnen Platten 2 bzw. zwischen zwei benachbarten Platten 2 kann gemäß der zuvor diskutierten Ausführungen die Anordnung mehrerer Kugeln 4 vorgesehen sein, die erfindungsgemäß wenigstens zwei verschiedene Durchmesser aufweisen. Abbildung 1b visualisiert dies unter Weglassung der Platten 2. Es gibt somit zumindest Kugeln einer ersten Gruppe mit einem großen Durchmesser und Kugeln einer zweiten Gruppe mit einem kleineren Durchmesser, wobei die Kugeln aus der Gruppe mit dem größeren bzw. größten Durchmesser aller Kugeln vorgesehen ist, um ein Maß für die MTF zu bestimmen und die Kugeln mit einem kleineren als dem größten Durchmesser vorgesehen sind, um die geometrische Abbildungsgenauigkeit bzw. die Verzerrung der Tomographen oder der zu Grunde liegenden Auswertealgorithmen zu bestimmen. Die MTF kann erfindungsgemäß ebenso anhand des wenigstens einen Rohres 3 bestimmt werden, ggfs. ausschließlich oder in Kombination mit Kugeln.

Unabhängig von der Form eines Prüfkörpers 1 kann es vorgesehen sein, dass von den kleineren Kugeln, die zur Bestimmung der Verzerrung vorgesehen sind, eine größere Anzahl vorhanden ist als von den größeren / größten Kugeln.

Bei einem kugelförmigen Prüfkörper, wie er hier dargestellt ist, kann es vorgesehen sein, dass wenigstens eine der größeren / größten Kugel pro Oktant des kugelförmigen Prüfkörpers vorhanden ist.

Die Abbildung 1a zeigt hier in der Außenansicht einen solchen Prüfkörper 1, der im wesentlichen Kugelgestalt aufweist und somit einen Kopf eines Menschen repräsentiert, der beispielsweise in einer später folgenden Untersuchung oder Behandlung mit dem Tomographen erfasst werden soll.

Diese Kugelform ist hier annähernd dadurch gebildet, dass ausgehend von einer mittleren Platte mit Kreisquerschnitt und größten Durchmesser beidseits an diese Platte nach oben und unten weitere Platten mit aufeinanderfolgend stufig kleiner werdenden Durchmesser gestapelt sind, so dass sich nach oben und unten der Durchmesser des Prüfkörper stufenweise verringert. Es ergibt sich so eine angenäherte Kugelform mit einem hier beispielhaft angegebenen Durchmesser von 17 cm.

Selbstverständlich sind hier jegliche anderen Maße möglich, sowie auch andere Formgestaltungen des Prüfkörpers, um andere Körperteile zu simulieren.

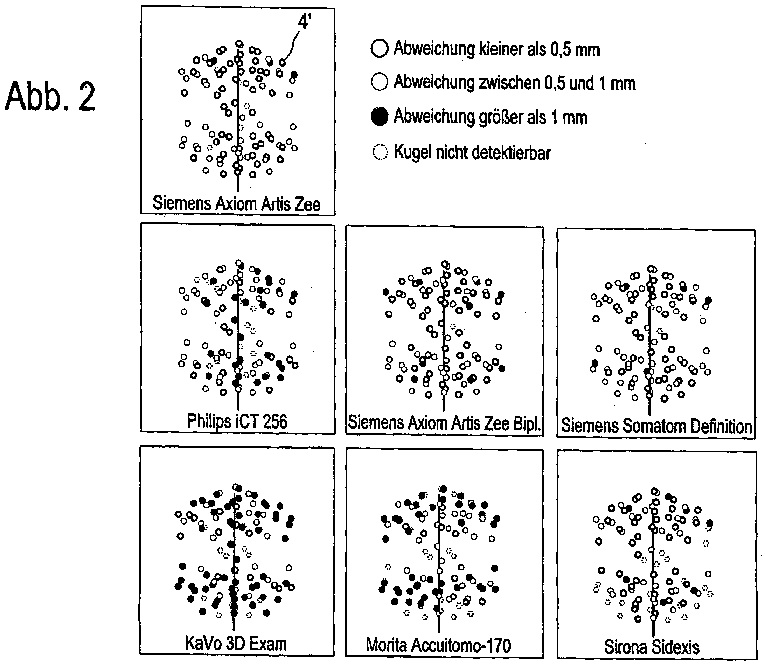

Die Abbildung 2 zeigt 3D-Darstellungen, die gebildet sind aus den einzelnen Schichtbildern eines Bildstapels, wobei in dieser jeweiligen Darstellung die Lage der einzelnen Kugeln durch deren jeweilige bildliche Wiedergabe repräsentiert ist.

Die Abbildung 2 zeigt hier für verschiedene Tomographen bzw. Hersteller derselben die Aufnahmen, die mit demselben Prüfkörper 1 erfasst bzw. berechnet wurden. Hier sind die einzelnen Kugeln bzw. deren bildliche Wiedergabe 4 im Bildstapel dahingehend untersucht worden, wie groß die Abweichung der bildlichen Repräsentation einer Kugel 4 gegenüber der tatsächlichen Sollposition im Prüfkörper ist, wobei die Größe der festgestellten Abweichung hier farbig markiert ist.

Erkennbar wird hier, dass unterschiedliche Tomographen, obwohl sie denselben Prüfkörper erfassen, verschiedene Abweichungen und somit Abbildungsfehler bei der bildlichen Wiedergabe des Prüfkörpers erzeugen.

Es besteht demnach die Möglichkeit mit ein und demselben Prüfkörper auch bildliche Wiedergaben verschiedener Tomographen in Kenntnis dieser Abweichungen aneinander zu matchen, beispielsweise um mit verschiedenartigen Tomographieverfahren bei einer späteren Anwendung zum Beispiel bei diagnostische Untersuchungen oder einer Therapie den tatsächlichen Körperteil zu untersuchen bzw. zu behandeln.

So steht beispielsweise die Möglichkeit tomographische Aufnahmen eines Magnetresonanz-Tomographen zu matchen d.h. zu überlagern mit den Röntgenaufnahmen eines Computertomographen, wenn nämlich einerseits zur Bestimmung der geometrischen Verzerrung des Computertomographen ein solcher Prüfkörper zum Einsatz kommt, der die Möglichkeit erschließt, die Kugeln in den einzelnen Röntgen-Schichtbildern wiederzugeben, und andererseits mit einem Prüfkörper einen MRT zu prüfen, wobei in diesem Prüfkörper statt beispielsweise metallischer Kugeln Kunststoffkugeln oder zumindest nicht magnetische / nicht magnetisierbare Kugeln eingesetzt werden. Hier ist vorgesehen dass die beiden verschiedenen Prüfkörper lediglich aus unterschiedlichen Materialien hinsichtlich der Platten und Kugeln ausgebildet sind, jedoch gleiche Geometrie, insbesondere mit gleichen Fertigungstoleranzen aufweisen

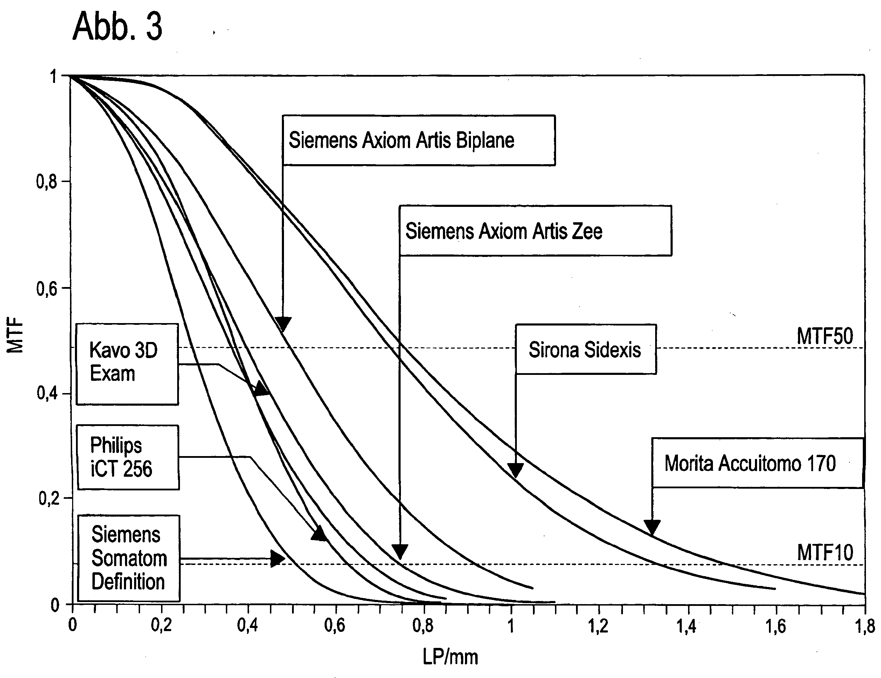

Die Abbildung 3 zeigt weiterhin, dass die Möglichkeit besteht, anhand der größeren bzw. größten Kugeln und/oder des wenigstens einen Rohres 3 die Modulations-Übertragungsfunktion mittels desselben Prüfkörpers bei unterschiedlichen Tomographen zu überprüfen. Hier können die Ergebnisse der Abbildungen 2 und 3 aus den gleichen Messserien ermittelt werden.

Die Abbildung 3 repräsentiert hier für die verschiedenen geprüften Tomographen jeweils die Modulations-Übertragungs-Funktion anhand der dargestellten Grafen, welche die MTF gegenüber dem Radius darstellt. Es ist bevorzugt erfindungsgemäß vorgesehen, dass die MTF so ermittelt wird, wie dies im eingangs beschriebenen allgemeinen Teil der Beschreibung erwähnt ist, insbesondere mit dem dort beschriebenen Überabtastungsprinzip.

Auch hier zeigt sich, dass bei der Verwendung desselben Prüfkörpers, wie in der Abbildung 1 dargestellt, sich unterschiedliche Modulation-Übertragungs-Funktion der verschiedenen Tomographen ergeben. Diese verschiedenen Tomographen mit den unterschiedlichen Modulationsübertragungsfunktion können demnach miteinander verglichen und somit anwendungsspezifisch ausgewählt werden.

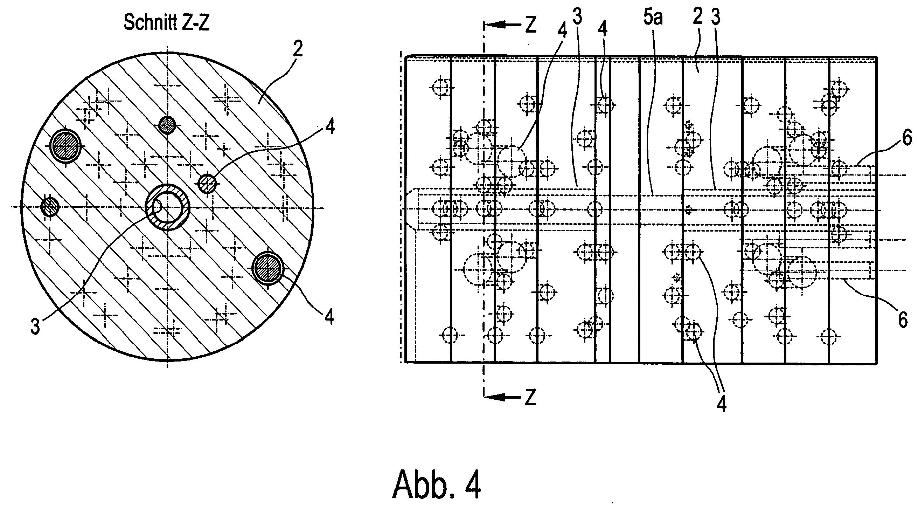

Die Figur 4 zeigt in zwei Ansichten einen zylindrischen Prüfkörper, der in axialer Richtung aus mehreren Platten 2 zusammengesetzt ist. Die linke Schnittdarstellung zeigt in einer Platte sowohl die darin angeordneten Kugeln 4 von verschiedenem Durchmesser als auch - hier mittig - in den kreisförmigen Platten 2 wenigstens ein Rohr 3, z.B. aus Metall wie Aluminium oder Magnesium. Der Vorteil ist hier, dass sich das Rohr 3 durch mehrere Platten 2 hindurch erstreckt und so in mehreren Schichtbildern dasselbe kreisringförmige Abbild liefert, so dass in diesen mehreren Schichtbilder die MTF immer anhand desselben Kriterium geprüft werden kann.

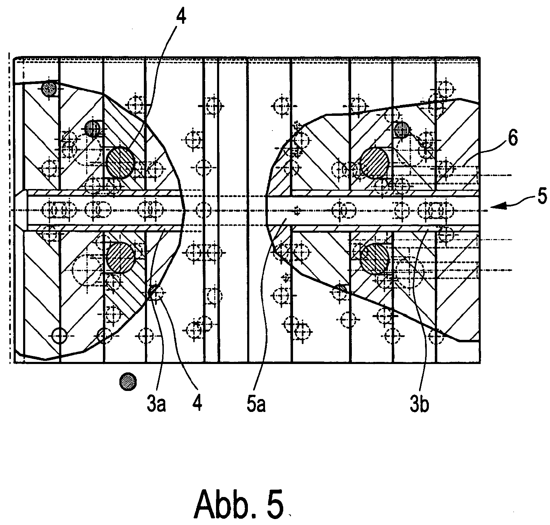

Diese Figur 4 zeigt in der rechten Darstellung auch eine mögliche Weiterbildung mit mehreren zusätzlichen Stäben 6, die gestrichelt dargestellt sind und alle auf das rechtsseitige stabförmige Hohlprofil 3 zulaufen. Die zusätzlichen Stäbe sind nur um das rechtsseitige Hohlprofil herum angeordnet und bevorzugt dort auch nur über einen Teilbereich von dessen Länge, insbesondere ausgehend von einer Deckplatte des Stapels. Auch Figur 5 zeigt dies als mögliche gestrichelte Weiterbildung.

Figur 5 zeigt eine Ausführungsvariante, in welcher ein hier beispielsweise zylindrischer Prüfkörper einen inneren Kanal 5 aufweist, in welchem zwei axial mit Abstand hintereinander liegende Rohre 3a und 3b eingeführt sind. Zwischen den Rohren 3a und 3b ergibt sich durch den Abstand ein innerer freier Kanalbereich 5a, der nicht von Rohrmaterial, insbesondere also nicht von Metall umgeben ist und in den (hier nicht dargestellt) z.B. eine Sensorvorrichtung oder ein anderes Gerät eingeführt werden kann, um zusätzliche Messwerte zu den Schichtbilder zu erfassen, wie z.B. die Strahlendosis, die zur Anwendung kam. Auch die rechte Darstellung von Figur 4 zeigt diesen freien Kanalbereich 5a.

Ein wesentlicher erfindungsgemäßer Vorteil liegt darin, dass sowohl die Modulationsübertragungsfunktion als auch ein Maß für die geometrischen Verzerrungen aus demselben Datensatz gewonnen werden kann, der mit ein und demselben Prüfkörper bei der tomographischen Aufnahme erstellt wird, ggfs. zusätzlich mit Erfassung einer Strahlendosis oder sonstiger Messwerte.

Nicht dargestellt ist es, mit einem Prüfkörper der erfindungsgemäßen Art ebenso das Rauschleistungsspektrum, sowie das Signal-Rauschverhältnis zu überprüfen. Hierbei kann auf grundsätzlich im Stand der Technik bekannte Algorithmen zurückgegriffen werden, jedoch unter Einsatz des erfindungsgemäßen Prüfkörpers.

Allgemein kann es in einer Weiterbildung auch vorgesehen sein, dass ein Prüfkörper der im allgemein Teil oder im Ausführungsteil beschriebenen Art in einen weiteren Körper, insbesondere in einen Prüfkörper ebenso der vorbeschriebenen Art, jedoch mit einem inneren freien Volumen eingesetzt wird. Hierdurch kann ein modulares System geschaffen werden.

## Patentansprüche

1. Prüfkörper zur Überprüfung der Übertragungseigenschaften von Tomographen, insbesondere radiologischen Tomographen, der mehrere zu einem Stapel verbundene Platten aufweist, in welchem benachbarte Platten (2) einander kontaktieren und der Kugeln (4) aus wenigstens einem zu den Platten (2) verschiedenen Material aufweist **dadurch gekennzeichnet, dass** er Kugeln (4) von wenigstens zwei verschiedenen Durchmessern aufweist, wobei die jeweiligen Kugeln (4) in und/oder zwischen wenigstens einigen der Platten (2) angeordnet sind und wobei am/im Prüfkörper, bevorzugt in dem Stapel wenigstens ein stabförmiges Hohlprofil, insbesondere wenigstens ein Rohr mit Kreisquerschnitt angeordnet ist.

2. Prüfkörper nach Anspruch 1, **dadurch gekennzeichnet, dass** die Platten (2) durch ein stabförmiges Hohlprofil, welches die Platten durchdringt, verbunden sind,

3. Prüfkörper nach einem der vorherigen Ansprüche, **dadurch gekennzeichnet, dass** in wenigstens einem der Hohlprofile wenigstens eine Sensorvorrichtung angeordnet ist, insbesondere eine Sensorvorrichtung zur Erfassung der bei einer Überprüfung aufgetretenen Strahlungsdosis.

4. Prüfkörper nach einem der vorherigen Ansprüche, **dadurch gekennzeichnet, dass** in dem Stapel wenigstens ein Kanal angeordnet ist, in welchem wenigstens ein Hohlprofil angeordnet ist, insbesondere austauschbar angeordnet ist.

5. Prüfkörper nach Anspruch 4, **dadurch gekennzeichnet, dass** in dem wenigstens einen Kanal zwei in axialer Richtung mit Abstand hintereinander liegende Hohlprofile, insbesondere Rohre angeordnet sind,

6. Prüfkörper nach Anspruch 5, **dadurch gekennzeichnet, dass** in einem Abstandsbereich zweier Hohlprofile in dem Kanal wenigstens eine Sensorvorrichtung angeordnet ist, insbesondere eine Sensorvorrichtung zur Erfassung der bei einer Überprüfung aufgetretenen Strahlungsdosis.

7. Prüfkörper nach einem der vorherigen Ansprüche, **dadurch gekennzeichnet, dass** seine äußere Form zumindest im Wesentlichen einem menschlichen oder tierischen Körperteil nachempfunden ist.

8. Prüfkörper nach einem der vorherigen Ansprüche, **dadurch gekennzeichnet, dass** einige der Kugeln (4), insbesondere die Kugeln mit dem größten Durchmesser in Abhängigkeit der Größe eines Pixels oder Voxels der Aufnahmesensorik eines zu prüfenden Tomographen derart gewählt sind, dass deren Durchmesser dem 20-fachen bis 70-fachen dieser Größe entspricht.

9. Prüfkörper nach Anspruch 8, **dadurch gekennzeichnet, dass** der Kugeldurchmesser im Bereich von 5 bis 10mm liegt.

10. Prüfkörper nach einem der vorherigen Ansprüche, **dadurch gekennzeichnet, dass** einige der Kugeln (4), insbesondere die Kugeln mit einem kleineren als dem größten Durchmesser in Abhängigkeit der Größe eines Pixels oder Voxels der Aufnahmesensorik eines zu prüfenden Tomographen derart gewählt sind, dass deren Durchmesser dem 10-fachen bis 20-fachen dieser Größe entspricht.

11. Prüfkörper nach Anspruch 10, **dadurch gekennzeichnet, dass** der Kugeldurchmesser im Bereich von 1 bis 5 mm liegt.

12. Prüfkörper nach einem der vorherigen Ansprüche, **dadurch gekennzeichnet, dass** die Kontaktebenen, in denen die einander kontaktierenden Oberflächen benachbarter Platten (2) liegen,
a. bei bestimmungsgemäßer Anordnung in einem zu prüfenden Tomographen nicht parallel zur den Ebenen der von dem Tomographen oder einer externen Auswerteeinheit berechneten Schichtbilder eines Schichtbilderstapels liegen
b. abweichend von einer senkrechten Anordnung relativ zum stabförmigen Hohlprofil (3) liegen.

13. Verfahren zur Überprüfung der Übertragungseigenschaften eines Tomographen mit einem Prüfkörper (1), bei dem mit dem Tomographen eine Serie mehrerer Aufnahmen von dem Prüfkörper (1) erstellt wird und aus der Serie mittels eines Tomograhie-Algorithmus im Tomographen oder in einer Auswerteeinheit ein Stapel mehrerer Schichtbilder des Prüfkörpers (1) berechnet wird, **dadurch gekennzeichnet, dass** ein Prüfkörper (1) nach einem der vorherigen Ansprüche eingesetzt wird und für wenigstens einen Teil, bevorzugt alle Schichtbilder des Bildstapels anhand der Bildwiedergaben wenigstens eines stabförmigen Hohlprofils ein Maß für die Modulations-Übertragungsfunktion berechnet wird.

14. Verfahren nach Anspruch 13, **dadurch gekennzeichnet, dass** anhand der Bildwiedergaben der Querschnitte der Kugeln (4) mit einem kleineren Durchmesser, insbesondere mit einem kleineren als dem größten Durchmesser die jeweilige Abweichung einer Kugel (4) in der Bildwiedergabe von ihrer bekannten Sollposition berechnet wird oder die Abweichungen der Kugeln (4) in der Bildwiedergabe untereinander von ihren bekannten Sollabständen berechnet werden.

15. Verfahren nach Anspruch 13 oder 14, **dadurch gekennzeichnet, dass** anhand der Bildwiedergaben der Querschnitte wenigstens einiger der Kugeln (4), insbesondere der Kugeln mit dem größten Durchmesser ein Maß für die Modulations-Übertragungsfunktion berechnet wird.

16. Verfahren nach einem der vorherigen Ansprüche 13-15, **dadurch gekennzeichnet, dass** für die Berechnung eines Maßes der Modulationsübertragungsfunktion in wenigstens einem der Schichtbilder bevorzugt in allen Schichtbildern durch eine Bilderkennungssoftware oder durch manuelle Auswahl wenigstens eine Bildwiedergabe des Querschnitts einer Kugel (4) oder des Querschnittes wenigstens eines stabförmigen Hohlprofils (3) selektiert wird.

17. Verfahren nach einem der vorherigen Ansprüche 13-16, **dadurch gekennzeichnet, dass** für die Berechnung eines Maßes der Modulationsübertragungsfunktion alle Intensitätsmesswerte der eine bildliche Wiedergabe (4') wenigstens eines Querschnitts einer Kugel (4), insbesondere mit dem größten Durchmesser oder wenigstens eines stabförmigen Hohlprofils (3) repräsentierenden Pixel / Voxel gemittelt werden, die innerhalb eines vorgegebenen Toleranzintervalls gleichen radialen Abstand zum Mittelpunkt der bildlichen Wiedergabe (4') haben.

18. Verfahren nach Anspruch 17, **dadurch gekennzeichnet, dass** für die Abhängigkeit der gemittelten Intensitätsmesswerte vom Radius eine Funktion gebildet / gefittet wird.

19. Verfahren nach Anspruch 14, **dadurch gekennzeichnet, dass** die Größe der ermittelten Abweichungen der jeweiligen bildlichen Wiedergaben (4') der Kugeln (4) von den Sollwerten in den Schichtbildern oder in einer aus diesen zusammengesetzten 3D-Darstellung visualisiert wird, insbesondere durch farbige Markierung der Kugelbildwiedergaben (4') mit Farben, die von der Größe der Abweichung abhängen.

20. Verfahren nach einem der vorherigen Ansprüche 13 bis 19 , **dadurch gekennzeichnet, dass** mittels wenigstens einer Sensorvorrichtung, die in einem stabförmigen Hohlprofil oder zwischen zwei axial beabstandet hintereinander liegenden Hohlprofilen angeordnet ist, die bei der Überprüfung eingesetzte Strahlendosis gemessen wird.

21. Verfahren nach einem der vorherigen Ansprüche 13 bis 20, **dadurch gekennzeichnet, dass** ein Prüfkörper innerhalb eines Tomographen gehalten wird mittels einer Haltevorrichtung, die zumindest zum Teil in das Innere wenigstens eines stabförmigen Hohlprofils eingreift, insbesondere den Prüfkörper durch das Innere wenigstens eines stabförmigen Hohlprofils hindurch durchdringt.

## Claims

1. Test body for checking the transmission properties of tomographs, in particular radiological tomographs, which has a plurality of plates (2) connected to form a stack in which adjacent plates (2) contact each other, and which has spheres (4) made of at least one material different from the plates (2) **characterized in that** it has spheres (4) of at least two different diameters, wherein the respective spheres (4) are arranged in and/or between at least some of the plates (2), and wherein at least one rod-shaped hollow profile, in particular at least one tube with a circular cross section, is arranged on/in the test body, preferably in the stack.

2. Test body according to Claim 1, **characterized in that** the plates (2) are connected by a rod-shaped hollow profile that passes through the plates.

3. Test body according to either of the preceding claims, **characterized in that** at least one sensor device is arranged in at least one of the hollow profiles, in particular a sensor device for measuring the radiation dose that occurs during a test.

4. Test body according to one of the preceding claims, **characterized in that** at least one channel is arranged in the stack, in which channel at least one hollow profile is arranged, in particular arranged exchangeably.

5. Test body according to Claim 4, **characterized in that** two hollow profiles, in particular tubes, are arranged at a distance from each other in the axial direction in the at least one channel.

6. Test body according to Claim 5, **characterized in that** at least one sensor device, in particular a sensor device for measuring the radiation dose that occurs during a test, is arranged in the channel in a space between two hollow profiles.

7. Test body according to one of the preceding claims, **characterized in that** its outer shape corresponds at least substantially to a human or animal body part.

8. Test body according to one of the preceding claims, **characterized in that** some of the spheres (4), in particular the spheres with the largest diameter, are chosen depending on the size of a pixel or voxel of the recording sensors of a tomograph to be tested, such that their diameter corresponds to 20 to 70 times this size.

9. Test body according to Claim 8, **characterized in that** the sphere diameter lies in the range of 5 to 10 mm.

10. Test body according to one of the preceding claims, **characterized in that** some of the spheres (4), in particular the spheres with a diameter smaller than the largest diameter, are chosen depending on the size of a pixel or voxel of the recording sensors of a tomograph to be tested, such that their diameter corresponds to 10 to 20 times this size.

11. Test body according to Claim 10, **characterized in that** the sphere diameter lies in the range of 1 to 5 mm.

12. Test body according to one of the preceding claims, **characterized in that** the contact planes, in which the mutually contacting surfaces of adjacent plates (2) lie,
a) do not lie parallel to the planes of the slice images of a slice image stack calculated by the tomograph or an external evaluation unit when arranged as intended in a tomograph to be tested,
b) deviate from a perpendicular arrangement relative to the rod-shaped hollow profile (3).

13. Method for checking the transmission properties of a tomograph using a test body (1), in which a series of several recordings of the test body (1) is established with the tomograph, and a stack comprising a plurality of slice images of the test body (1) is calculated from the series by means of a tomography algorithm in the tomograph or in an evaluation unit, **characterized in that** a test body (1) according to one of the preceding claims is used, and a measure of the modulation transmission function is calculated for at least some slice images, preferably all the slice images, of the image stack, on the basis of the image reproductions of at least one rod-shaped hollow profile.

14. Method according to Claim 13, **characterized in that**, on the basis of the image reproductions of the cross sections of the spheres (4) with a smaller diameter, in particular with a diameter smaller than the largest diameter, the respective deviation of a sphere (4) in the image reproduction from its known desired position is calculated, or the deviations between one another of the spheres (4) in the image reproduction from their known desired separations are calculated.

15. Method according to Claim 13 or 14, **characterized in that** a measure of the modulation transmission function is calculated on the basis of the image reproductions of the cross sections of at least one of the spheres (4), in particular the spheres with the largest diameter.

16. Method according to one of Claims 13-15, **characterized in that**, for calculating a measure of the modulation transmission function in at least one of the slice images, preferably in all of the slice images, at least one image reproduction of the cross section of a sphere (4) or of the cross section of at least one rod-shaped hollow profile (3) is selected by image recognition software or by manual selection.

17. Method according to one of Claims 13-16, **characterized in that**, for calculating a measure of the modulation transmission function, all the intensity measurements of the pixels/voxels that represent a visual reproduction (4') of at least one cross section of a sphere (4), in particular with the largest diameter, or of at least one rod-shaped hollow profile (3), are averaged, which within a specified tolerance interval are at the same radial distance from the centre point of the visual reproduction (4').

18. Method according to Claim 17, **characterized in that** a function is formed/fitted for the dependency of the averaged intensity measurements on the radius.

19. Method according to Claim 14, **characterized in that** the magnitude of the determined deviations of the respective visual reproductions (4') of the spheres (4) from the desired values in the slice images, or in a 3D representation compiled from these, is visualized in particular by coloured marking of the sphere image reproductions (4') with colours that depend on the magnitude of the deviation.

20. Method according to one of Claims 13 to 19, **characterized in that** the radiation dose used in the test is measured by means of at least one sensor device which is arranged in a rod-shaped hollow profile or between two hollow profiles that lie one behind the other with an axial spacing.

21. Method according to one of Claims 13 to 20, **characterized in that** a test body is held inside a tomograph by means of a retaining device, which engages at least partially in the interior of at least one rod-shaped hollow profile, in particular extends through the test body via the interior of at least one rod-shaped hollow profile.

## Revendications

1. Corps de test destiné à tester les propriétés de transmission de tomographes, notamment de tomographes radiologiques, qui comporte une pluralité de plaques (2) reliées en un empilement dans lequel des plaques (2) adjacentes sont en contact les unes avec les autres et qui comporte des billes (4) constituées d'au moins un matériau différent de celui des plaques (2), **caractérisé en ce qu'**il comporte des billes (4) ayant au moins deux diamètres différents, dans lequel les billes (4) respectives sont disposées dans et/ou entre au moins certaines des plaques (2) et dans lequel au moins un profil creux en forme de tige, notamment au moins un tube de section transversale circulaire, est disposé sur/dans le corps de test, de préférence dans l'empilement.

2. Corps de test selon la revendication 1, **caractérisé en ce que** les plaques (2) sont reliées par l'intermédiaire d'un profil creux en forme de tige qui passe à travers les plaques.

3. Corps de test selon l'une quelconque des revendications précédentes, **caractérisé en ce qu'**au moins un dispositif détecteur est disposé dans au moins l'un des profils creux, notamment un dispositif détecteur destiné à détecter la dose de rayonnement s'étant produite lors d'un test.

4. Corps de test selon l'une quelconque des revendications précédentes, **caractérisé en ce qu'**au moins un canal, dans lequel est disposé au moins un profil creux, est disposé, notamment de manière remplaçable, dans l'empilement.

5. Corps de test selon la revendication 4, **caractérisé en ce que** deux profils creux, notamment des tubes, sont disposés dans l'au moins un canal, de manière espacée l'un à l'arrière de l'autre dans la direction axiale.

6. Corps de test selon la revendication 5, **caractérisé en ce qu'**au moins un dispositif détecteur, notamment un dispositif détecteur destiné à détecter la dose de rayonnement se produisant lors d'un test, est disposé dans le canal, dans une zone d'espacement de deux profils creux.

7. Corps de test selon l'une quelconque des revendications précédentes, **caractérisé en ce que** sa forme extérieure ressemble au moins sensiblement à une partie corporelle humaine ou animale.

8. Corps de test selon l'une quelconque des revendications précédentes, **caractérisé en ce que** certaines des billes (4), notamment les billes ayant le diamètre le plus important, sont sélectionnées en fonction de la taille d'un pixel ou d'un voxel du système détecteur d'acquisition d'un tomographe à tester, de manière à ce que leur diamètre corresponde à 20 fois à 70 fois ladite taille.

9. Corps de test selon la revendication 8, **caractérisé en ce que** le diamètre des billes se situe dans la gamme de 5 à 10 mm.

10. Corps de test selon l'une quelconque des revendications précédentes, **caractérisé en ce que** certaines des billes (4), notamment les billes ayant un diamètre inférieur au diamètre le plus important, sont sélectionnées en fonction de la taille d'un pixel ou d'un voxel du système détecteur d'acquisition d'un tomographe à tester, de manière à ce que leur diamètre corresponde à 10 fois à 20 fois ladite taille.

11. Corps de test selon la revendication 10, **caractérisé en ce que** le diamètre des billes se situe dans la gamme de 1 à 5 mm.

12. Corps de test selon l'une quelconque des revendications précédentes, **caractérisé en ce que** les plans de contact dans lesquels se trouvent les surfaces en contact les unes avec les autres des plaques adjacentes (2),
a. sont placés, pour un agencement correspondant à un fonctionnement normal dans un tomographe à tester, de manière non parallèle aux plans des vues en coupe, calculées par le tomographe ou par une unité d'évaluation externe, d'un empilement de vues en coupes,
b. sont placés d'une manière qui s'écarte d'un agencement perpendiculaire par rapport à un profil creux (3) en forme de tige.

13. Procédé destiné à tester les propriétés de transmission d'un tomographe au moyen d'un corps de test (1), dans lequel une série d'une pluralité d'acquisitions du corps de test (1) est réalisée à l'aide du tomographe et un empilement d'une pluralité de vues en coupe du corps de test (1) est calculé dans le tomographe ou dans une unité d'évaluation à partir de ladite série au moyen d'un algorithme de tomographie, **caractérisé en ce qu'**un corps de test (1) selon l'une quelconque des revendications précédentes est utilisé, et **en ce que**, pour au moins une partie, de préférence pour la totalité des vues en coupe de l'empilement de vues, une mesure de la fonction de transfert de modulation est calculée sur la base des reproductions d'images d'au moins un profil creux en forme de tige.

14. Procédé selon la revendication 13, **caractérisé en ce que**, sur la base des reproductions d'images des sections transversales des billes (4) ayant un diamètre inférieur, notamment un diamètre inférieur au diamètre le plus important, l'écart respectif d'une bille (4) dans la reproduction d'image, par rapport à sa position nominale connue, est calculé, ou les écarts des billes (4) les unes par rapport aux autres dans la reproduction d'image, et par rapport à leurs espacements nominaux connus, sont calculés.

15. Procédé selon la revendication 13 ou 14, **caractérisé en ce qu'**une mesure de la fonction de transfert de modulation est calculée sur la base des reproductions d'images des sections transversales d'au moins certaines des billes (4), notamment des billes ayant le diamètre le plus important.

16. Procédé selon l'une quelconque des revendications 13 à 15 précédentes, **caractérisé en ce que**, pour calculer une mesure de la fonction de transfert de modulation dans au moins l'une des vues en coupe, de préférence dans toutes les vues en coupe, au moins une reproduction d'image de la section transversale d'une bille (4) ou de la section transversale d'au moins un profil creux (3) en forme de tige est sélectionnée par un logiciel de reconnaissance d'image ou par sélection manuelle.

17. Procédé selon l'une quelconque des revendications 13 à 16, **caractérisé en ce que**, pour calculer une mesure de la fonction de transfert de modulation, la totalité des valeurs de mesure d'intensité des pixels/voxels représentant une reproduction d'image (4') d'au moins une section transversale d'une bille (4), ayant notamment le diamètre le plus important, ou d'au moins un profil creux en forme (3) de tige, sont déterminées, lesquelles valeurs présentent les mêmes distances radiales, à un intervalle de tolérance prédéterminé près, par rapport au centre de la reproduction d'image (4').

18. Procédé selon la revendication 17, **caractérisé en ce qu'**une fonction est construite /ajustée pour la dépendance des valeurs de mesure d'intensité déterminées par rapport au rayon.

19. Procédé selon la revendication 14, **caractérisé en ce que** l'importance des écarts déterminés des reproductions d'images respectives (4') des billes (4) par rapport aux valeurs nominales dans les vues en coupe ou dans une représentation tridimensionnelle composée à partir de celles-ci, est visualisée, notamment par un marquage coloré des reproductions d'images de billes (4') à l'aide de couleurs qui dépendent de l'importance de l'écart.

20. Procédé selon l'une quelconque des revendications 13 à 19 précédentes, **caractérisé en ce que** la dose de rayonnement introduite lors de l'examen est mesurée au moyen d'au moins un dispositif détecteur qui est disposé dans un profil creux en forme de tige ou entre deux profils creux disposés l'un derrière l'autre de manière axialement espacée.

21. Procédé selon l'une quelconque des revendications 13 à 20 précédentes, **caractérisé en ce qu'**un corps de test est maintenu à l'intérieur d'un tomographe au moyen d'un dispositif de maintien qui vient en prise, au moins en partie, à l'intérieur d'au moins un profil creux en forme de tige, et qui traverse notamment le corps de test à travers l'intérieur d'au moins un profil creux en forme de tige.
